Europäisches Patentamt

European Patent Office

Office européen des brevets

(19)

(11) Publication number: 0 235 834
A1

# (12) EUROPEAN PATENT APPLICATION

(21) Application number: 87200109.4

(51) Int. Cl.4: **A61B 6/00**

(22) Date of filing: 26.01.87

(30) Priority: 30.01.86 NL 8600215
20.03.86 NL 8600711

(43) Date of publication of application:
09.09.87 Bulletin 87/37

(84) Designated Contracting States:
DE FR GB NL

(71) Applicant: N.V. Philips' Gloeilampenfabrieken
Groenewoudseweg 1
NL-5621 BA Eindhoven(NL)

(72) Inventor: Welberg, Antonius Johannus
Gerardus
c/o INT. OCTROOIBUREAU B.V. Prof.
Holstlaan 6
NL-5656 AA Eindhoven(NL)
Inventor: Hoeks, Antonius Johannes
Ludovicus M.
c/o INT. OCTROOIBUREAU B.V. Prof.
Holstlaan 6
NL-5656 AA Eindhoven(NL)
Inventor: A-Tjak, Anton Marcel
c/o INT. OCTROOIBUREAU B.V. Prof.
Holstlaan 6
NL-5656 AA Eindhoven(NL)
Inventor: van Maanen, Cornelis Hubertus
Berend H.
c/o INT. OCTROOIBUREAU B.V. Prof.
Holstlaan 6
NL-5656 AA Eindhoven(NL)

(74) Representative: Scheele, Edial François et al
INTERNATIONAAL OCTROOIBUREAU B.V.
Prof. Holstlaan 6
NL-5656 AA Eindhoven(NL)

(54) Surgical X-ray diagnostic machine.

(57) In the case of known surgical X-ray diagnostic machines the control desk (2) is positioned in such a way that many users operate the apparatus in an ergonomically unfavourable posture because of their different heights. It is proposed to provide a surgical X-ray diagnostic machine in which reciprocal orientations, dimensions and positions of parts of the machine and the centre of rotation of a C-arc (8) are adapted to an ergonomically determined position of the control desk (2).

FIG.1

## Surgical X-ray diagnostic machine.

The invention relates to a surgical X-ray diagnostic machine equipped with an X-ray source and an X-ray image detector which are coupled opposite each other to a C-arc, the said C-arc being adjustably mounted on a stand and the said surgical X-ray diagnostic machine also comprising a control desk integrated into it.

A surgical X-ray diagnostic machine of this kind is known from Diagnostic Imaging, August 1985, pages 66-67. The mobile X-ray diagnostic machine shown there consists of a mobile stand and a monitor trolley. The stand contains an X-ray source and an X-ray image detector which are coupled opposite each other to a C-arc, and a control desk which comprises the X-ray and image processing controls. The monitor trolley contains monitors and image processing equipment. It is known that in order to position a stand of this kind with respect to, for example, a patient to be examined, a C-arc as mentioned above can rotate around horizontal and vertical axes and can translate in parallel to a horizontal and a vertical axis. These degrees of freedom of the C-arc make it possible, for example, to irradiate a patient to be examined with X-radiation at various angles without moving him or her. After detection of X-rays by the X-ray image detector and the processing of signals by the image processing equipment, an irradiated part of a body is visualised with the aid of a monitor on the monitor trolley.

A disadvantage of the known mobile surgical X-ray diagnostic machine is that the control desk is positioned in such a way that a great many users operate the machine in an ergonomically unfavourable- posture because of their different heights. This operation comprises, on the one hand, the operation of, for example, knobs present on the desk and the reading off of the symbols on this and, on the other hand, the operation of the C-arc and the stand by means of, for example, handgrips.

The object of the invention is to provide a surgical X-ray diagnostic machine with ergonomic operation for a relatively large number of users of different heights.

For this purpose, a surgical X-ray diagnostic machine of the kind mentioned in the preamble is characterised in that reciprocal orientations, dimensions and positions of parts of the machine and a centre of rotation of the C-arc are adapted to an ergonomically determined position of the control desk. When the control desk is raised for ergonomic reasons in order to ensure ready accessibility to, for example, a patient to be examined, the X-ray diagnostic machine will be adapted to this, in the case of normal C-arc dimensions, by making a C-arc slide block (i.e. a slide block with which the C-arc is fixed to the stand) suitable for a higher position of the C-arc, or by increasing the radius of the C-arc. Increasing the radius of the C-arc has consequences, on the one hand, for the distance from the centre of an object to be examined to the X-ray image intensifier and, on the other hand, for the distance from this centre to the centre of rotation of the C-arc. The centre of rotation of the C-arc here is the centre of rotation in what is termed an orbital movement, i.e. a rotational movement of the C-arc in its own plane. Minimising the first distance reduces distortions in an image of the object and results in a desired minimal geometrical distortion. Minimising the second distance prevents the object moving out of the centre of a beam emitted by the X-ray source when the C-arc is orbiting around the centre of rotation. When the radius of the C-arc is increased, the position of the centre of mass of the whole assembly of X-ray source, X-ray image intensifier and C-arc also changes. This position is important because it is desirable to be able to operate a surgical X-ray diagnostic machine manually. In particular, this operation comprises the translation and rotation of the C-arc.

An embodiment of a surgical X-ray diagnostic machine according to the invention, in which the X-ray image detector contains a detachable 9 inch tube, is characterised in that a distance between the centre of an object and a centre of rotation of the C-arc lies in an interval of 80 to 100 mm. At the two most important positions of the machine when it is in operation, namely the X-ray source and X-ray image intensifier in line with each other either vertically or horizontally during the orbital movement, the centre of the object will, within reasonable limits, now lieclose to the point of intersection of the X-ray beams which vary with respect to these positions. It should be mentioned that the centre of the object here is defined as a point of intersection of central rays of X-ray beams at the above-mentioned vertical and horizontal position, respectively.

A further embodiment of a surgical X-ray diagnostic machine according to the invention, in which the X-ray image detector contains a detachable 9 inch tube, is characterised in that C-arc has an external or an internal radius of approximately 557 mm or approximately 462 mm, respectively, and in order to balance the X-ray source, the X-ray image detector and/or the C-arc is weighted and that the distance from the centre of mass of the whole assembly of X-ray source, X-ray image detector and C-arc to the point of intersection of the axes of rotation of the C-arc is minimised. As a result, it is possible to move the C-arc according to its degrees of freedom with relatively little force.

A further embodiment of a surgical X-ray diagnostic machine according to the invention, in which the X-ray image detector contains a detachable 9 inch tube, is characterised in that the distance from the focus of the X-ray source to a bearing floor for the equipment is minimised. This makes is possible to place the X-ray source relatively easily under, for example, a patient table.

A further embodiment of a surgical X-ray diagnostic machine according to the invention, in which the X-ray image detector contains a detachable 9 inch tube, is characterised in that the control desk contains various segments, each of which corresponds to a control function. This increases the clear and orderly arrangement of the control desk and promotes ergonomic operation of the machine.

A further embodiment of a mobile surgical X-ray diagnostic machine, in which the X-ray image detector contains a detachable 9 inch tube, is characterised in that each of the segments is at least partly detachable. As a result, a control desk contains precisely just as many segments, i.e. control functions, as a user wishes, which again increases the clear and orderly arrangement of the control desk and promotes the ergonomic operation of the machine.

The invention will be further explained on the basis of a mobile X-ray diagnostic unit partly shown in figure 1 and figure 2 and a control desk shown in figure 3.

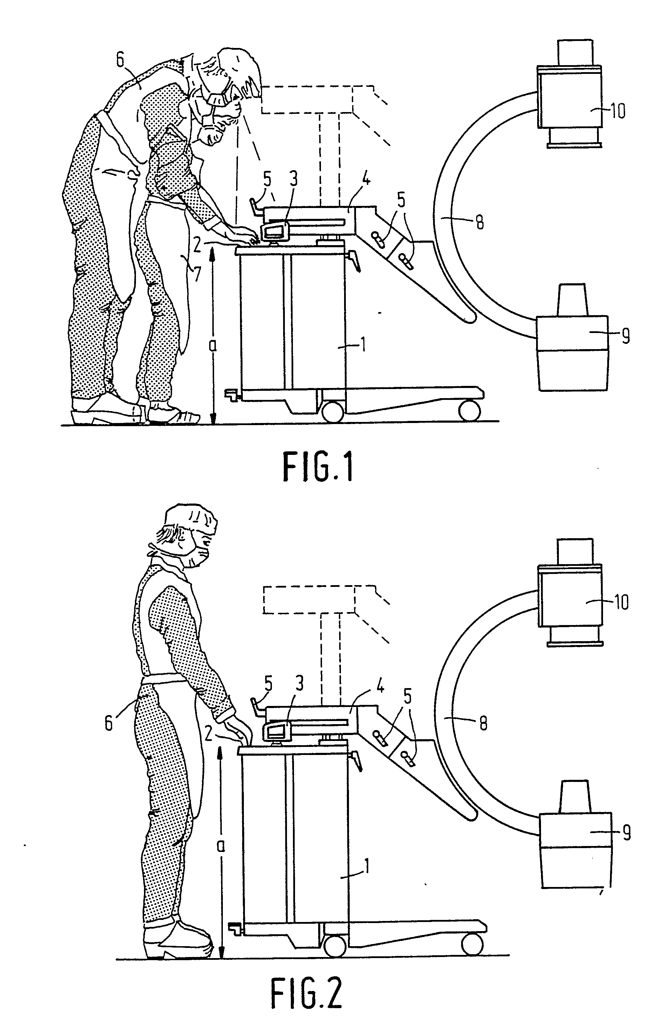

Figure 1 shows a mobile stand 1, a control desk 2 with handgrip 3 and a C-arc arm 4 with levers 5. Dashed lines indicate the highest position of the C-arc arm 4 if a C-arc 8 connected to it with X-ray source 9 and X-ray image intensifier 10, which contains a 9 inch tube, is translated maximally in the vertical direction. At a distance a = 75 cm, which is normal for the known X-ray diagnostic machines, a man 6 with a height of 185 cm adopts a very ergonomically unfavourable posture when operating the machine. When the C-arc arm is in the highest position (dashed lines) the man 6 may even bump his head. At the distance a = 75 cm the posture of a woman 7 with a height of 151 cm is more or less acceptable.

Part of an X-ray diagnostic machine is drawn in figure 2, in which respect the reference numbers correspond to those in figure 1. By positioning the control desk 2 at an ergonomically determined level (a = 90 cm) the posture of a man 6 with a height of 185 cm is ergonomically acceptable. A woman with a height of 151 cm (not drawn) would also operate the machine in an ergonomically acceptable position. Raising the control desk makes it necessary to adapt the levers to the height, for example, if the accessibility of the levers on the C-arc arm is to remain optimum. As a result of this, either the C-arc arm must be longer if the C-arc stays the same, or the C-arc must become bigger if the C-arc arm stays the same. It has been found particularly advantageous to design the C-arc with an external or internal radius of 557 mm or 462 mm, respectively. In that case the distance between the centre of an object and the centre of rotation of the C-arc lies in the interval of 80 to 100 mm. If the C-arc is made larger, the machine may become unbalanced. In order to balance it the X-ray source, X-ray image detector and/or the C-arc can be weighted. For example, the whole assembly can be brought back into balance by attaching lead to the X-ray image detector.

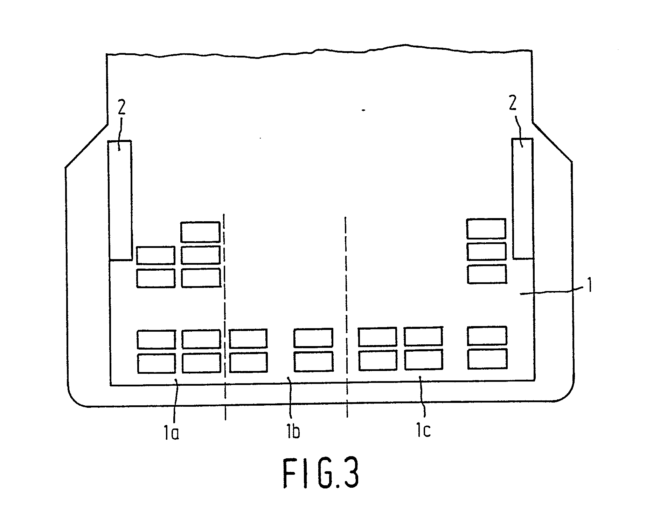

A control desk 1 with handgrips 2 is drawn in figure 3. The control desk is designed in such a way that each of the parts 1a, 1b and 1c corresponds to a control function or a collection of control functions which belong logically together, for example the C-arc movement, X-ray dose rate control, etc.

## Claims

1. Surgical X-ray diagnostic machine equipped with an X-ray source and an X-ray image detector which are coupled opposite each other to a C-arc, the said C-arc being adjustably mounted on a stand and the said surgical X-ray diagnostic machine also comprising a control desk integrated into it, characterised in that reciprocal orientations, dimensions and positions of parts of the machine and the centre of rotation of the C-arc are adapted to an ergonomically determined position of the control desk.

2. Surgical X-ray diagnostic machine according to claim 1, in which the X-ray image detector contains a detachable 9 inch tube, characterised in that a distance between the centre of an object and the centre of rotation of the C-arc lies in an interval of 80 to 100 mm.

3. Surgical X-ray diagnostic machine according to claim 1 or 2, in which the X-ray image detector contains a detachable 9 inch tube, characterised in that a C-arc has an external or an internal radius of approximately 557 mm or approximately 462 mm, respectively, and in order to balance the X-ray source, the X-ray image detector and/or the C-arc is weighted, and in that the distance from the centre of mass of the whole assembly of X-ray source, X-ray image detector and C-arc to the point of intersection of the axes of rotation of the C-arc is minimised.

4. Surgical X-ray diagnostic machine according to claim 1, 2 or 3, in which the X-ray image detector contains a detachable 9 inch tube, characterised in that the distance between the focus of the X-ray source and a bearing floor for the equipment is in an interval of approximately 250 to 350 mm.

5. Surgical X-ray diagnostic machine according to claims 1, 2, 3 or 4, in which the X-ray image detector contains a detachable 9 inch tube, characterised in that the control desk contains various segments, each of which corresponds to a control function.

6. Surgical X-ray diagnostic machine according to claim 5, characterised in that at least some of the segments are separately detachable.

FIG.1

FIG.2

FIG.3

European Patent
Office

**EUROPEAN SEARCH REPORT**

Application number

EP 87 20 0109

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 549 885 (S.E.L. ANDERSSON) * Abstract; column 2, lines 34-75; column 4, lines 1-8; figures 1,2,6 * | 1 | A 61 B 6/00 |
| A | | 5 | |
| Y | FR-A-2 061 116 (SIEMENS AG) * Page 1, lines 28-34; page 2, lines 20-39; figures * | 1 | |
| A | GB-A-2 098 440 (PHILIPS) * Abstract; page 1, lines 45-51,114-121; page 2, lines 35-49; page 3, lines 31-107; figures 2,3 * | 1,3,4 | |
| A | IBM TECHNICAL DISCLOSURE BULLETIN, vol. 28, no. 2, July 1985, pages 747-750, New York, US; "Adjustable terminal table" * Page 747, lines 1-3; page 750, lines 1,2 * | 1 | TECHNICAL FIELDS SEARCHED (Int. Cl.4) A 61 B A 47 B |
| A | FR-A-2 406 425 (SIEMENS AG) * Page 3, lines 2-6; figures 1-3 * | 1,6 | |

The present search report has been drawn up for all claims

| Place of search | Date of completion of the search | Examiner |
|---|---|---|
| THE HAGUE | 08-05-1987 | RIEB K.D. |